(19) Europäisches Patentamt
European Patent Office
Office européen des brevets

(11) Publication number: **0 292 169 B1**

# (12) EUROPEAN PATENT SPECIFICATION

(45) Date of publication of patent specification: **23.11.94**

(51) Int. Cl.[5]: **C07H 15/203**, C07H 23/00, C12Q 1/34, G01N 33/58

(21) Application number: **88304173.3**

(22) Date of filing: **09.05.88**

(54) **Substrates for B-galactosidase.**

(30) Priority: **21.05.87 US 52328**

(43) Date of publication of application:
**23.11.88 Bulletin 88/47**

(45) Publication of the grant of the patent:
**23.11.94 Bulletin 94/47**

(84) Designated Contracting States:
**BE CH DE ES FR GB IT LI NL SE**

(56) References cited:
**EP-A- 0 060 793**
**EP-A- 0 146 866**
**EP-A- 0 249 357**
**DE-A- 3 323 245**
**GB-A- 2 014 154**

**PATENT ABSTRACTS OF JAPAN vol. 12, no. 172 (C-497)(3019), 21 May 1988; & JP-A-62278998 (TOYOBO CO. LTD.) 03.12.1987**

(73) Proprietor: **MILES INC.(an Indiana corp.)**
**511 Benedict Avenue**
**Tarrytown, New York 10591-5097 (US)**

(72) Inventor: **Hwang, Deng Ruey**
**52 Tarryhill Road**
**Tarrytown New York 10591 (US)**
Inventor: **Scott, Mary Ellen Ann**
**90 High Moutain Road**
**Ringwood New Jersey 07456 (US)**

(74) Representative: **Wain, Christopher Paul et al**
**A.A. THORNTON & CO.**
**Northumberland House**
**303-306 High Holborn**
**London WC1V 7LE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 292 169 B1

## Description

This invention relates to liposomal immunoassays, and particularly to substrates for immunoassays utilizing β-galactosidase.

In the assay of digoxin and other clinically important low concentration analytes which may be present in serum, especially human blood serum, using β-galactosidase-encapsulated liposomes, it is important to utilize an extremely sensitive chromogenic substrate in order to achieve the essential assay dynamic range. This is especially true when such an assay is performed on an automated analysis instrument such as, for example, a TECHNICON RA-1000 clinical chemistry analyzer. (TECHNICON RA-1000 is a registered trademark of Technicon Instruments Corporation, Tarrytown, NY.)

There are several known substrates for β-galactosidase, including: 2-methoxy-4-(2-nitrovinyl)-phenyl-β-D-galactopyranoside, which was disclosed as a substrate for the assay of β-galactosidase (C.T. Yuen, Analytica Chimica Acta, 163, (1982) 195-204); resorufin-β-D-galactopyranoside, which was disclosed as a fluorogenic substrate for β-galactosidase (Analytica Chimica Acta, 163, (1984) 67-72); E-1-1 (1-deoxylactulosyl)-2-lysine, which was disclosed as a substrate for determining the activity of β-galactosidase in the intestinal tract of mice (J. of Chromatography, 278, (1983) 275-282; and two high molecular weight substrates for β-D-galactosidase, each containing β-D-[3-H]-galactopyranosyl moieties linked through an aliphatic bridge to either poly-2-lysine or polymeric dialdehyde, which were disclosed by R. Madhan et al. (Enzyme, 25 (1980) 127-131).

EP-A-146866 describes the use of sulphonphthaleinyl-β-D-galactosides as substrates for the determination of β-D-galactosidase. EP-A-249357 describes enzyme immunoassays employing enzyme labels capable of converting a substrate to a coloured product, and discusses the coversion of nitrophenyl-β-D-galactoside (p- and/or o-) to nitrophenol by β-galactosidase. GB-A-2014154 discloses a β-galactosidase which is produced by Penicillium multicolor.

The most common substrate for β-galactosidase currently in use is O-nitrophenyl-β-D-galactopyranoside (ONPG) which is preferred for its relatively fast enzyme turnover rate, good stability in aqueous buffers and the relative ease by which it can be synthesized or commercially obtained, ONPG has a significant disadvantage, though, in that it has a relatively small molar absorptivity upon enzyme-catalyzed hydrolysis. Assays for digoxin and other clinically important low concentration analytes which utilize β-galactosidase encapsulated liposomes require a more sensitive substrate to achieve the essential dynamic range.

We have now found some new compounds useful as substrates for β-galactosidase which when so used are of increased sensitivity and are stable in aqueous buffers.

According to the invention, there is provided a compound useful as a substrate for β-galactosidase, which compound has the general formula:

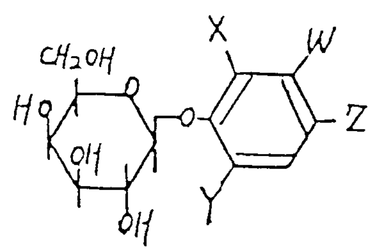

wherein X is a halogen; Y is halogen, lower alkyl or hydrogen; W is hydrogen or lower alkyl; and Z is nitro.

For the purpose of this invention, the term "lower" means that the group contains from 1 to 6 carbon atoms; the term "alkyl" refers to a straight or branched chain hydrocarbon containing no unsaturation, e.g. methyl, ethyl, isopropyl, 2-butyl, neopentyl, n-hexyl; the term "halogen" refers to fluorine, chlorine, bromine and iodine; the term "hapten" refers to any substance which does not stimulate antibody formation but reacts selectively in vitro with an antibody; and the term "serum" refers to any physiological fluid, such as blood.

The compounds of this invention can be prepared in the following manner (the substituents W, X, Y and Z being as defined above unless indicated otherwise).

A compound of the formula (II)

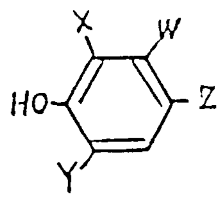

(II)

is reacted with a galactopyranoside having the formula (IIa)

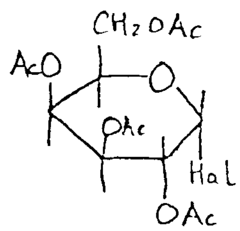

(IIa)

wherein Hal is halogen and Ac is acetyl, under anhydrous conditions in the presence of $K_2CO_3$ and acetone for a period of 36 hours, to form a compound of formula (III)

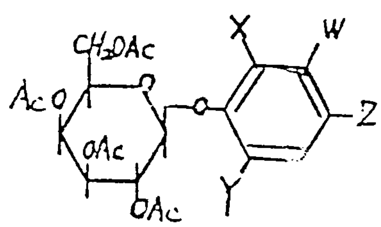

(III)

Compound III is then reacted under anhydrous conditions in the presence of $CH_3ONa$ and methanol for 36 hours to form a compound I.

Compounds useful as substrates for β-galactosidase are those in which X and Y are halogen, more preferably chlorine, W is hydrogen and Z is nitro (2,6-dichloro-4-nitrophenyl-β-D-galactopyranoside).

The most preferred composition of this invention, 2-chloro-4-nitrophenyl-β-D-galactopyranoside (CLNPG), is one in which X is halogen, preferably chlorine, Y and W are each hydrogen and Z is nitro.

The compositions of this invention are useful as chromogenic substrates for β-galactosidase and are suitable for use in assays, especially immunoassays utilizing β-galactosidase. Typical applications for which the new substrate are exceptionally well suited include the use of antibody- or hapten-β-galactosidase conjugates in homogeneous and heterogeneous immunoassays in manual kit format or on analytical instruments such as the TECHNICON RA-1000 clinical chemistry analyzer or a TECHNICON CHEM-1 clinical chemistry analyzer. (TECHNICON CHEM-1 is a trademark of Technicon Instruments Corporation, Tarrytown, NY.). Protocols for such assays are conventional in the art and are known to the skilled artisan.

The hapten liposome immunoassay protocol adapted for use on the TECHNICON RA-1000 clinical chemistry analyzer is typical of such protocols and is summarized below:

| Samples | Reagent A | | Reagent B |
|---|---|---|---|
| 30ul | CLNPG substrate | 160µl | liposome 35 |
| | Complement | 80µl | |
| | PC | 40µl | |
| | Buffer | 22µl | |
| | Antobody | 48µl | |
| | 4 minutes, 37°C. | | |
| | 5 minutes, 37°C. | | |

wherein Complement refers to serum protein, PC refers to phosphocholine, a stabilizer, and the hapten-modified liposome 35 component is prepared by the following film deposition process. The membrane components consist of lecithin, sphingomyelin, tocopherol, dicetyl phosphate, cholesterol and hapten phospholipid conjugate. The membrane components are dissolved in chloroform solvent and evaporated on the inner surface of a one liter reaction vessel. The film is then hydrated with an aqueous buffer solution containing $\beta$-galactosidase, which is the entrapped enzyme marker, in the same reaction vessel. Liposomes spontaneously form in high yield. Separation of reagent liposomes from unentrapped enzyme is accomplished by ultracentrifugation.

The assay protocol involves the addition of sample and Reagent A into the reaction cuvette, incubation for 4 minutes, addition of Reagent B, and a second incubation of 5 minutes. Actual analyte concentrations are determined from a functionally linear standard curve from the calibrators.

The protocol outline above was followed in Examples V and VI discussed below.

The following working Examples describe experiments which were performed in developing the present invention. Standard commercially available reagent grade chemicals were used whenever possible. These Examples are to be considered illustrative of the present invention and should not be interpreted as limiting its scope.

In the accompanying drawings, which are presented to further describe the invention and to assist in its understanding through clarification of its various aspects,

FIGS. 1 and 2 are Lineweaver and Burk plots for a preferred compound of the present invention; and

FIG. 3 is a digoxin immunoassay - antibody titration curve comparing the performance of the above preferred compound of the present invention with that of the preferred prior art compound.

EXAMPLE 1

A. Synthesis of 2-chloro-4-nitrophenyl-2,3,4,6-tetra-o-acetyl-$\beta$-D-galactopyranoside

To a solution of 2-chloro-4-nitrophenol, commercially available from Aldrich Chemical Company of Mulwaukee, Wisconsin, 43.39 g (0.25 mM) in 150 ml of acetone, was added 34.56 g (0.25 mM) of anhydrous potassium carbonate to form a suspension. To this suspension was added 96.69 g (0.24 mM) of acetobromo-alpha-D-galactose to form a reaction mixture, which was heated to about 55 ° C for 36 hours. The reaction mixture was monitored by thin layer chromatography (EM Silica gel 60 F254 in ethyl acetate/benzene [3/7 by volume]). The desired product ($R_f$ = 0.41) is detectable by short ultraviolet light as a brown spot after spraying with methanol/sulfuric acid (9/1 by volume) and heating at about 125 ° C for 5 minutes. When the desired product was found, the reaction mixture was then cooled, filtered and evaporated with a water aspirator. The residue was dissolved in 40 ml of chloroform and washed with 1N cold sodium hydroxide and then with water. The combined organic layer was dried (using anhydrous sodium sulfate) and evaporated to dryness. The product was then crystallized from hot methanol to yield 91.94 g of product (73%), having a melting point of 147-149 ° C. Elemental Analysis: % C Actual 47.60 (Theoretical 47.67); % H 4.45 (4.41); %N 2.68 (2.78); % CL 6.91 (7.05); % O 38.02 (38.10).

B. Synthesis of 2-chloro-4-nitrophenyl-$\beta$-D-galactopyranoside (CLNPG)

1.0026 g (1.99 mM) of the product of Example IA was suspended in dry methanol and a catalytic amount of anhydrous sodium methoxide (12 mg) was added to form a reaction mixture which was maintained at 4 ° C for 36 hours. The reaction mixture was monitored by thin layer chromotography [EM

silica gel 60 F254 in chloroform/methanol (6/1 by volume], after which the reaction mixture was neutralized with acetic acid and the solvent evaporated. The residue was dissolved in hot methanol and a small amount (40 mg) of activated charcoal was added. The resulting mixture was then filtered to remove the charcoal and the filtrate was allowed to crystallize overnight. The crystals were then rinsed with ether and dried to yield 0.58 g of product (87%), having a melting point of 213-215 °C and $R_f$ of 0.18. Elemental analysis % C, 42.65 (42.93); % H 4.06 (4.21); % N 3.92 (4.17); % Cl 10.82 (10.56); % O 38.17 (38.13).

EXAMPLE II

The molar absorptivity ($\epsilon_{max}$) of the phenolic precursors of several substrates of this invention were measured and calculated according to the following formula:

$$\epsilon_{max} = \frac{A}{c \times b}$$

wherein A is the absorbance; b is the path length through the sample; and c is the concentration of solute.

The molar absorptivity of the phenolic precursor of ONPG, the most commonly used prior art substrate for $\beta$-galactosidase was likewise measured and calculated for comparative purposes. The aim was to identify phenolic precursors which would provide enhanced molar absorptivity to that of ONPG.

The results are set out in Table I.

TABLE I

| Phenol | Molar Absorptivity ($\epsilon_{max}$) |
|---|---|
| bromocresol purple | 50.7 (585 nm, pH 8.5) |
| 2-chloro-4-nitrophenol (CLNP) | 16.9 (405 nm, pH 7.5) |
| 2,6-dichloro-4-nitrophenol | 15.9 (405 nm, pH 7.5) |
| 2-chloro-4,6-dinitro phenol | 11.5 (405 nm, pH 7.5) |
| 2,4-dinitro-6-methyl phenol | 11.0 (405 nm, pH 7.5) |
| 2,4-dinitro phenol (DNP) | 10.7 (405 nm, pH 7.5) |
| metacresol purple | 8.4 (575 nm, pH 8.5) |
| 2-chloro-6-nitrophenol | 4.6 (405 nm, pH 7.5) |
| orthonitro phenol (ONP) | 3.0 (405 nm, pH 7.5) |

These results clearly show that the substrates of the present invention display relatively high molar absorptivity, which relates to the chromogenic characteristics of the substrate upon chemical and enzymatic hydrolysis, especially when compared to the preferred prior art substrate.

EXAMPLE III

One object of the present invention was to provide a new substrate for $\beta$-glactosidase which has a higher enzymatic rate than the preferred prior art composition.

The mechanism of one substrate enzymatic reaction can be represented as follows:

$$S + E \underset{K_{-1}}{\overset{K_1}{\rightleftharpoons}} ES \xrightarrow{K_2} E + P$$

wherein S is substrate, E is enzyme, P is product, $K_1$ and $K_2$ are the rate constants for the forward reaction, and $K_{-1}$ is the rate constant for the reverse reaction.

The detailed discussion of the chemical reaction kinetics is presented in "Principles of Enzymatic Analysis" edited by Hans Ulrich Bergmeyer in collaboration with Karlfried Gamehn 1978, Verlag Chemie.

Various kinetic parameters of the enzyme reaction were measured including Km, Vmax and Kp.

Km is the Michalis constant. It expresses the substrate concentration at which the reaction rate has half of its maximum value.

$$K_m = \frac{K_{-1} + K_2}{K_1}$$

$K_m$ is by no means an absolute constant, but depends on pH, temperature, effectors, buffers, etc. The reaction conditions should therefore always be specific in connection with the Km value.

The reaction rate can be expressed as

$$v = \frac{V}{1 + Km/S}$$

The most commonly used method for calculation of Km is that of Lineweaver and Burk.

$$\frac{1}{v} = \frac{Km}{V} \times \frac{1}{[S]} + \frac{1}{V}$$

This is the equation of a straight line (l/v plotted against l/[S]), corresponds to y = ax + b.

Insertion of y = o gives ax = -b, -x = b/a. In this case for l/$\nu$ = o (i.e., intercept on the abscissa), [S] = Km.

If x = o, then y = b. In this case: for l/[S] = o (i.e., intercept on the ordinate), l/v = l/V.

Both important characteristics of an enzyme (Km and V max) are thus obtainable in a single operation

Kp is the turnover number which is easily obtained if V max ($V_m$) and enzyme concentration (Ec) are known. Kp is expressed as Kp = Vm/Ec.

Calculated values of Km, V max and Kp for CLNPG of the present invention, and, by way of comparison, for BCPG, are based on experimental data plotted in Figs. 1 and 2 respectively, and appear in Table II as do values for ONPG and DNPG which are available from the literature.

Table II

| Substrate | Km(mM) | V max (ΔA/Min) | Kp(Vm/Ec,$Min^{-1}$) |
|---|---|---|---|
| 2-chloro-4-nitrophenyl-$\beta$-D-galactopyranoside (CLNPG) | 0.20 | 0.13 | $7.40 \times 10^4$ |
| bromocresol purple-$\beta$-D-galactopyranoside (BCPG) | 8.0 | 0.11 | $3.10 \times 10^2$ |
| orthonitrophenyl-$\beta$-D-galactopyranside (ONPG) | 0.17 | 0.03 | $1.82 \times 10^5$ |
| 2,4-dinitro-phenyl-$\beta$-D-galactopyranoside (DNPG) | 0.22 | 0.13 | $2.20 \times 10^5$ |

The advantage of the preferred substrate of this invention CLNPG (2-chloro-4-nitrophenyl-$\beta$-D-galactopyranoside) over ONPG, the preferred prior art substrate, in terms of V max which relates to the maximum enzymatic rate, is evident from the above. While the Km and Kp parameter of both compositions

are similar, the V max of CLNPG is at least four times that of ONPG. DNPG, while showing good kenetic parameters is unstable in aqueous environment.

EXAMPLE IV

The enzymatic response of 2-chloro-4-nitrophenyl-β-D-galactopyranoside (CLNPG) was measured and compared with that of ortho-nitrophenyl-β-D-galactopyranoside (ONPG) by the following free enzyme protocol performed on a TECHNICON RA-1000 clinical chemistry analyzer:

15 µl of known concentration of β-galactosidase in buffer (1mg/ml) was added to 300 µl of substrate (2.5mM in buffer). The buffer used was 50mM Tris HCl and 150mM NaCl and 5mM $MgCl_2$ (pH 7.5). The enzymatic reaction product was measured at 405nM after 30 seconds and the ΔA 405 nm/min for three different concentrations of β-galactosidase was recorded for each substrate. The results are shown in Table III.

Table III

| ß-galactosidase concentration | Δ 405/min. (CLNPG) | Δ 405/min. (OPNG) | Ratio (CLNPG/ONPG) |
|---|---|---|---|
| 0 | .0007 | .0003 | -- |
| 1/2000 | ..0427 | .0077 | 5.5 |
| 1/1000 | 0'.0923 | 0.0159 | 5.8 |
| 1/200 | 0.4555 | 0.0795 | 5.7 |

EXAMPLE V

The enzymatic response rate of 2-chloro-4-nitrophenyl-β-D-galactopyranoside (CLNPG) was measured and compared with that of orthonitrophenyl-β-D-galactopyranoside (ONPG) by the following β-galactosidase encapsulated liposome protocol performed on a TECHNICON RA-1000 clinical chemistry analyzer:

15 µl of liposome was added to 300 µl of substrate (2.5 mM in buffer) and mixed for 30 seconds. The buffer used was 0.05 M Tris HCl and 150 mM NaCl and 5mM $MgCl_2$ (pH 7.5). The enzymatic rate of the mixture was then measured at 405nm (-T). For chemical lysis, the identical mixture was prepared but 0.1% Triton X-100 was substituted for the buffer. The enzymatic rate of the mixture was measured at 405nm (+T). The 405nm/min [(+T) - (-T)] for three different concentrations of liposome was recorded for each substrate. The results are shown in Table IV.

Table IV

| ß-galactosidase-encapsulated liposome concentration (1/20 Dilution) | Δ405/min (CLNPG) | Δ405 nm/min (ONPG) | CLNPG/ ONPG |
|---|---|---|---|
| 0.1 | 0.0180 | 0.0030 | 6.0 |
| 0.5 | 0.0915 | 0.0138 | 6.6 |
| 1.0 | 0.1803 | 0.0279 | 6.5 |

EXAMPLE VI

A digoxine liposome immunoassay was performed by using the above-mentioned protocol to compare the utilizing of CLNPG and ONPG. The antibody titration curve, as shown in Fig. 3, clearly illustrates that the performance of CLNPG was superior to that of ONPG, the former offering a four-fold increase in assay sensitivity over the latter for a wider range of antibody.

It is apparent from the above that the use of the preferred substrate of this invention in both free enzyme and β-galactosidase-encapsulated liposome assays allows for an assay at least 5 times as sensitive as current prior art assays.

## Claims

1. A compound useful as a substrate for β-galactosidase, which compound has the general formula:

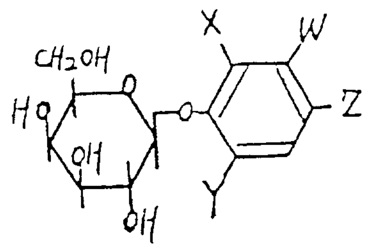

wherein X is a halogen; Y is halogen, lower alkyl or hydrogen; W is hydrogen or lower alkyl; and Z is nitro.

2. A compound according to claim 1, wherein X is chlorine or bromine, Y is chlorine or methyl and W is methyl.

3. A compound according to claim 1, wherein X is chlorine; Y is hydrogen and W is hydrogen.

4. A compound according to claim 1, wherein X and Y are each chlorine and W is hydrogen.

5. A compound according to claim 1, wherein Y is lower alkyl and W is hydrogen.

6. An assay using β-galactosidase as a signal generating marker wherein there is used as the substrate a compound as claimed in any of claims 1 to 5.

## Patentansprüche

1. Verbindung, die als ein Substrat für β-Galaktosidase nützlich ist und die allgemeine Formel

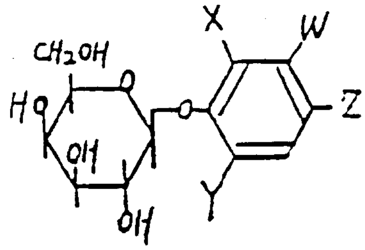

hat, worin X ein Halogen ist; Y ist Halogen, Niederalkyl oder Wasserstoff; W ist Wasserstoff oder Niederalkyl und Z ist Nitro.

2. Verbindung nach Anspruch 1, worin X Chlor oder Brom ist, Y Chlor oder Methyl und W Methyl ist.

3. Verbindung nach Anspruch 1, worin X Chlor ist; Y ist Wasserstoff und W ist Wasserstoff.

4. Verbindung nach Anspruch 1, worin X und Y jeweils Chlor sind und W Wasserstoff ist.

5. Verbindung nach Anspruch 1, worin Y Niederalkyl und W Wasserstoff ist.

6. Test, bei dem $\beta$-Galaktosidase als Signal-erzeugender Marker verwendet wird, wobei als das Substrat eine Verbindung nach einem der Ansprüche 1 bis 5 verwendet wird.

## Revendications

1. Composé utile en tant que substrat de la $\beta$-galactosidase, lequel composé possède la formule générale :

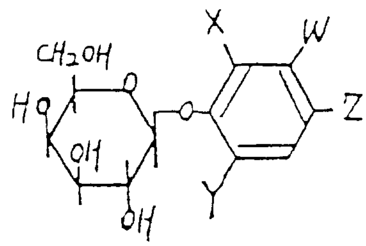

dans laquelle X représente un atome d'halogène, Y représente un atome d'halogène, un groupement alkyle inférieur ou l'atome d'hydrogène, W représente l'atome d'hydrogène ou un alkyle inférieur et Z représente le groupement nitro.

2. Composé selon la revendication 1, dans lequel X représente l'atome de chlore ou l'atome de brome, Y représente l'atome de chlore ou le groupement méthyle et W représente le groupement méthyle.

3. Composé selon la revendication 1, dans lequel X représente l'atome de chlore, Y représente l'atome d'hydrogène et W représente l'atome d'hydrogène.

4. Composé selon la revendication 1, dans lequel X et Y représentent chacun l'atome de chlore et W représente l'atome d'hydrogène.

5. Composé selon la revendication 1, dans lequel Y représente un groupement alkyle inférieur et W représente l'atome d'hydrogène.

6. Dosage utilisant de la $\beta$-galactosidase en tant que marqueur générant un signal, dans lequel on utilise, en tant que substrat, un composé selon l'une quelconque des revendications 1 à 5.

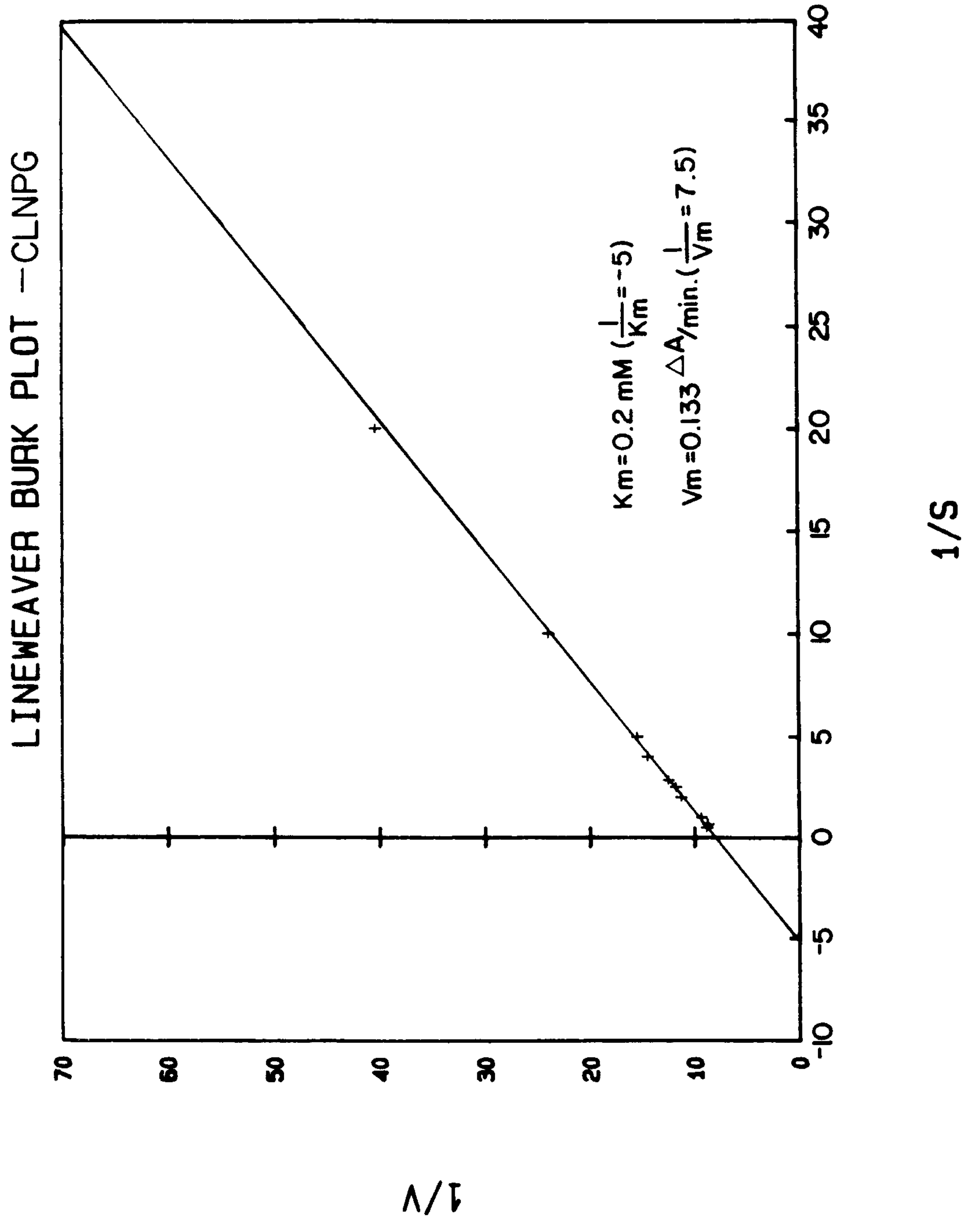
LINEWEAVER BURK PLOT —CLNPG
Km=0.2 mM (1/Km = -5)
Vm=0.133 ΔA/min. (1/Vm = 7.5)
1/V
1/S
0
10
20
30
40
50
60
70
-10
-5
0
5
10
15
20
25
30
35
40

FIG.1

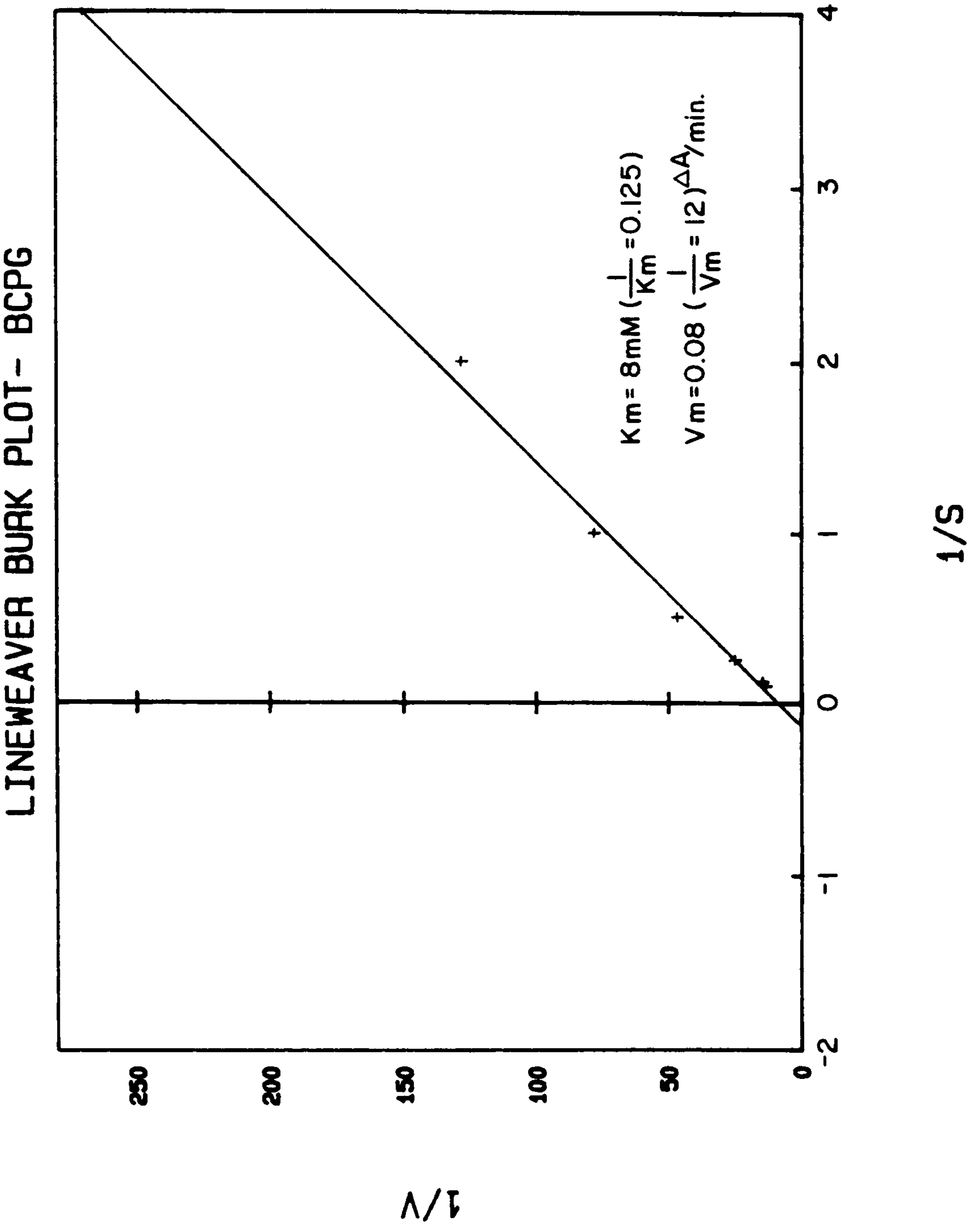
LINEWEAVER BURK PLOT- BCPG
Km= 8mM (1/Km =0.125)
Vm=0.08 (1/Vm = 12) ΔA/min.
1/V
250
200
150
100
50
0
-2
-1
0
1
2
3
4
1/S

FIG.2

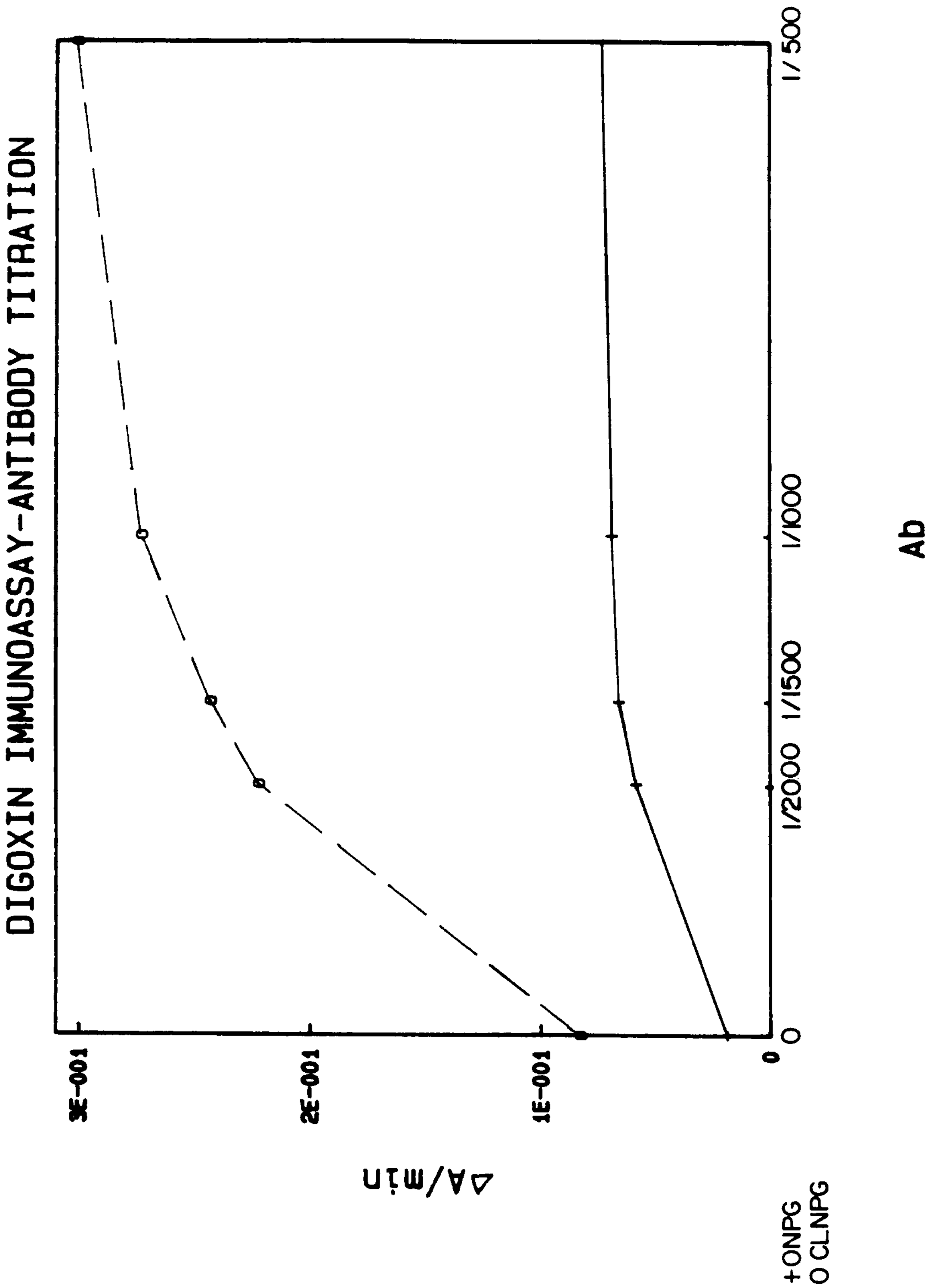
DIGOXIN IMMUNOASSAY-ANTIBODY TITRATION
ΔA/min
3E-001
2E-001
1E-001
0
0
1/2000
1/1500
1/1000
1/500
Ab
+ONPG
O CL.NPG

FIG.3